# EUROPEAN PATENT SPECIFICATION

(11) **EP 2 205 557 B1**
(45) Date of publication and mention of the grant of the patent: **29.02.2012**
(21) Application number: 08831702.9
(22) Date of filing: 19.09.2008
(51) Int. Cl.: C07C 319/04, C07C 323/56, C07C 323/54, C07C 323/61, C07B 45/06

(54) **METHOD FOR PRODUCING BETA-MERCAPTOCARBOXYLIC ACIDS**
VERFAHREN ZUR HERSTELLUNG VON BETA-MERCAPTOCARBONSÄUREN
PROCEDE DE PRODUCTION D'ACIDES BETA-MERCAPTOCARBOXYLIQUES

(30) Priority: 20.09.2007 JP 2007243279
(43) Date of publication of application: 14.07.2010
(73) Proprietor: Showa Denko K.K., Tokyo 105-8518 (JP)
(72) Inventor: SHIBUYA, Akira, Kawasaki-shi Kanagawa 210-0865 (JP); AOKI, Hidemasa, Kawasaki-shi Kanagawa 210-0865 (JP)
(74) Representative: Strehl Schübel-Hopf & Partner
(86) International application number: PCT/JP2008/067580
(87) International publication number: WO 2009/038232

(56) References cited:
- EP-A- 0 208 323
- EP-A- 0 831 084
- EP-A- 1 149 813

## Description

### TECHNICAL FIELD

The present invention relates to a method for efficiently producing β-mercaptocarboxylic acid, β-mercaptocarboxylic acid ester, β-mercaptoamide, β-mercapto aldehyde and β-mercaptoketone (hereinafter, these compounds are collectively referred to as "β-mercaptocarboxylic acids") by allowing a starting material, which corresponds to respective target products, selected from an α,β-unsaturated carboxylic acid group consisting of (α,β-unsaturated carboxylic acid, α,β-unsaturated carboxylic acid ester, α,β-unsaturated amide, α,β-unsaturated aldehyde and α,β-unsaturated ketone to react with hydrogen sulfides selected from a group consisting of hydrogen sulfide, sulfide salt and hydrosulfide salt.

### BACKGROUND ART

Conventionally, mercapto compounds have been widely used as synthetic materials for various medicinal products and agricultural chemicals. Particularly, effectiveness of β-mercaptocarbonyl compound as antioxidative agent is highly acknowledged and the compound is being industrially used as a stabilizer for polymer compounds (Japanese Patent Application Laid-Open No. 2003-252918). The reaction described in Acta Chimica Scandinavica 1951, 5, 690-698, in which reaction diethylamine is used in large excess, however, includes problems that reaction time is long, resulting in low productivity and that the reaction method requires equipment for recovering diethylamine in industrial-scale and inexpensive production process.

It has been reported that Michael addition reaction using an acrylic acid as α,β-unsaturated carboxylic acid proceeds by using hydrogen sulfide in the presence of diethylamine (Acta Chimica Scandinavica 1951, 5, 690-698).

In addition, it has been reported that β-mercaptopropionic acid which is a β-mercapto compound can be synthesized by allowing acrylic acid to react with sodium hydrosulfide in the presence of sodium hydroxide present in large excess (Japanese Patent Application Laid-Open No. 2001-187778). This method in which, however, also has a problem that 5 equivalent amount of sodium hydroxide based on the substrate is required in order to suppress side-reaction, which requires neutralization of excessive alkali with acid and further requires treatment of inorganic salts massively generated in the reaction, which hinders industrial-scale production.

As for other reactions, a method for synthesizing β-mercaptopropionic acid methyl ester through reaction between acrylic acid methyl ester and sodium hydrosulfide with carbon disulfide used as auxiliary reagent has been reported (Great Britain Patent No. 1358019). In the reaction described in this document, it is proposed to use carbon disulfide for the purpose of suppressing side reaction. The method, however, is not very useful in industrial sense, in that carbon disulfide having high inflammability is used and that purification steps after the reaction are cumbersome.

The synthesis of mercapto-3 propionic acid is disclosed in EP-0831084 involving the reaction between H₂S and acrylic acid in the presence of a solid basic catalyst.

Further, Japanese Patent Application Laid-Open No. 2001-354643 (US Patent No. 6544936) and Japanese Patent Application Laid-Open No. 2001-354644 (US Patent No. 6472354), describe methods for producing sulfurized olefins by use sulfur compounds and hydrogen sulfide to sulfurize olefins in the presence of solid acid catalyst such as zeolites. Although these documents include description about mercaptans as intermediate product, the final target products in the documents are not mercapto compounds which are the target compound of the present invention but organic sulfides, disulfides and polysulfides. The techniques in the documents try to reduce production of mercaptan to the minimum. Moreover, the documents describe that raw material olefin may be diluted with solvent. The examples of solvent mentioned in the documents are saturated aliphatic hydrocarbons such as methane, ethane and pentane, i.e., apolar solvents. Neither of the documents describes or suggests using a solvent which is compatible with water, a polar solvent, or effects of using such a solvent.

### DISCLOSURE OF INVENTION

The object of the present invention is to provide β-mercaptocarboxylic acids which are useful as raw materials for synthesis of pharmaceutical products and agricultural chemicals and as additives in polymer compounds, by using α,β-unsaturatedcarboxylic acids which are readily available as raw materials, in good yields, with high productivity and on an industrial scale.

As a result of intensive studies with a view to achieving the object, the inventors of the present invention have found out a method of using a reaction solvent compatible with water in producing β-mercaptocarboxylic acids through reaction between α,β-unsaturated carboxylic acids and hydrogen sulfides in the presence of solid acid catalyst such as zeolite, to thereby complete the invention.

In the present invention, a "solvent compatible with water" is a solvent in which water can dissolve at an amount 5% by mass or more at reaction temperature.

That is, the present invention relates to a production method of β-mercaptocarboxylic acids as described in the following [1] to [13].
[1] A method for producing β-mercaptocarboxylic acids, comprising using as reaction solvent a solvent which is compatible with water in a reaction between α,β-unsaturated carboxylic acids selected from a group consisting of α,β-unsaturated carboxylic acid, α,β-unsaturated carboxylic acid ester, α,β-unsaturated amide, α,β-unsaturated aldehyde and α,β-unsaturated ketone and at least one of hydrogen sulfides selected from a group consisting of hydrogen sulfide, sulfide salt and hydrosulfide salt in the presence of a solid acid catalyst (excluding silica gel), wherein the β-mercaptocarboxylic acids to be produced corresponds to the starting materials.
[2] The method for producing β-mercaptocarboxylic acids according to 1, wherein a mixed solvent of a solvent compatible with water and water is used as the reaction solvent.
[3] The method for producing β-mercaptocarboxylic acids according to 2, wherein the water content of the reaction solvent is within a range of 1 to 50 % by mass.
[4] The method for producing β-mercaptocarboxylic acids according to any one of 1 to 3, wherein the solvent compatible with water is a polar aprotic solvent or an alcohol.
[5] The method for producing β-mercaptocarboxylic acids according to 4, wherein the polar aprotic solvent is one or more selected from a group consisting of tetrahydrofuran(THF), N,N-dimethyl formamide(DMF), 1,3-dimethyl-2-imidazolidinone(DMI) and N-methyl-2-pyrrolidone(NMP).
[6] The method for producing β-mercaptocarboxylic acids according to 1, wherein the selected α,β-unsaturated carboxylic acid is α,β-unsaturated carboxylic acid or α,β-unsaturated carboxylic acid ester.
[7] The method for producing β-mercaptocarboxylic acids according to 1 or 6, wherein the α,β-unsaturated carboxylic acid is acrylic acid, methacrylic acid, crotonic acid, 2-pentenoic acid, fumaric acid, maleic acid, maleic anhydride, cinnamic acid, α-methyl cinnamic acid, (2'-methyl)cinnamic acid, (3'-methyl)cinnamic acid, (4'-methyl)cinnamic acid, (2',3'-dimethyl)cinnamic acid, (2',4'-dimethyl)cinnamic acid, (3',4'-dimethyl)cinnamic acid, (2'-hydroxy)cinnamic acid, (3'-hydroxy)cinnamic acid, (4'-hydroxy)cinnamic acid, (2',3'-dihydroxy)cinnamic acid, (2',4'-dihydroxy)cinnamic acid, (3',4'-dihydroxy)cinnamic acid, 2-hexenoic acid or 4-methyl-2-pentenoic acid.
[8] The method for producing β-mercaptocarboxylic acids according to 1 or 6, wherein the α,β-unsaturated carboxylic acid ester is methyl acrylate, ethyl acrylate, propyl acrylate, butyl acrylate, methyl methacrylate, ethyl methacrylate, propyl methacrylate, butyl methacrylate, methyl crotonate, ethyl crotonate, propyl crotonate, butyl crotonate, methyl 2-pentenate, ethyl 2-pentate, propyl 2-pentenate, or butyl 2-pentenate.
[9] The method for producing β-mercaptocarboxylic acids according to 1, wherein the α,β-unsaturated ketone is cyclopentenone, cyclohexenone, or cycloheptenone.
[10] The method for producing β-mercaptocarboxylic acids according to 1, wherein the solid acid catalyst is at least one kind selected from a group consisting of acidic ion exchange resin, zirconium compound, zeolite, double oxidesupported silca gel, hydrotalcite, attapulgite, kaolinite, alumina, hydroxyapatite, and heteropoly acid-supported catalyst.
[11] The method for producing β-mercaptocarboxylic acids according to 1, wherein the solid acid catalyst is zeolite.
[12] The method for producing β-mercaptocarboxylic acids according to 11, wherein the zeolite is molecular sieves 3A, molecular sieves Y-54, molecular sieves LZ-15, molecular sieves AZ-300, molecular sieves 4A, molecular sieves 5A, or molecular sieves 13X.
[13] The method for producing β-mercaptocarboxylic acids according to 1, wherein the sulfide salt or hydrosulfide salt is alkali metal salt, alkali earth metal salt, ammonium salt, amine salt having 12 or less carbon atoms.

The present invention provides a method for producing β-mercaptocarboxylic acids, through a reaction between α,β-unsaturated carboxylic acids as raw material selected from a group consisting of α,β-unsaturated carboxylic acid, α,β-unsaturated carboxylic acid ester, α,β-unsaturated amide, α,β-unsaturated aldehyde and α,β-unsaturated ketone and hydrogen sulfides selected from a group consisting of hydrogen sulfide, metal sulfide salt and metal hydrosulfide salt.

According to the production method of the present invention, β-mercaptocarboxylic acids can be obtained in high yields with high productivity, and the method, which can simplify purification process, is extremely useful as an industrial production method.

### BEST MODE FOR CARRYING OUT THE INVENTION

The present invention is characterized in that as reaction solvent, a solvent compatible with water is employed in the reaction between the α,β-unsaturated carboxylic acids and the hydrogen sulfides in the presence of solid acid catalyst such as zeolite to produce β-mercaptocarboxylic acids. Hereinafter, the present invention is specifically described.

### [α,β-unsaturated carboxylic acids]

As α,β-unsaturatedcarboxylic acids used as raw material for reaction in the method of the present invention, any one of α,β-unsaturated carboxylic acid, α,β-unsaturated carboxylic acid ester, α,β-unsaturated amide, α,β-unsaturated aldehyde and α,β-unsaturated ketone may be employed. Here, the term "α,β-unsaturated" means a state in which a carbon atom at the α position adjacent to carbonyl carbon(C=O) and a carbon atom at the β position adjacent to the α position form a double bond. Each hydrogen atom bonding to α- and β-carbons may be substituted with an alkyl group, cycloalkyl group, aryl group, aralkyl group, alkoxy group, carboxyl group, acyl group, alkoxycarbonyl group(ester), or acyloxy group. In a case where these substituents contain another carbonyl carbon, the α- and β-positions are based on the original unsaturated carbon. These substituents may be the same with or different from each other. These substituents may have other functional groups bonded thereon. For example, it may be an alkyl group (for instance 2-oxo-propyl group) having an oxo group.

In addition, compounds consisting of the α,β-unsaturated carboxylic acid having a carboxyl group bonded to its original β-position, such as maleic acid, fumaric acid and maleic anhydride are also included in α,β-unsaturated carboxylic acids used in the present invention. Further, α-carbon and β-carbon may have a cyclic structure by bonding through an alkylene group or the like. Such a cyclic structure may have a structure of a cyclic ketone where a carbonyl carbon is present on the ring or a structure containing nitrogen. Preferred among the above substituents are alkyl group and aryl group in consideration for availability as raw materials.

Examples of alkyl group include methyl group, ethyl group, n-propyl group, isopropyl group, n-butyl group, isobutyl group, 1-methylpropyl group, tert-butyl group, n-pentyl group, isopentyl group, 1-methylbutyl group, 2-methylbutyl group, 1-ethylpropyl group, n-hexyl group, isohexyl group, 1-methylpentyl group, 2-methylpentyl group, 3-methylpentyl group, 1-ethylbutyl group, and 2-ethylbutyl group. Preferred among them are methyl group, ethyl group, and isopropyl group.

Examples of cycloalkyl group include cyclopentyl group, cyclohexyl group, and cycloheptyl group. Preferred among them are cyclopentyl group and cyclohexyl group.

Examples of aryl group include phenyl group, tolyl group, xylyl group, and naphthyl group.

Examples of aralkyl group include benzyl group, and phenethyl group. Preferred among them are phenyl group and benzyl group.

Examples of alkoxy group include methoxy group and ethoxy group.

Examples of acyl group include formyl group, acetyl group, and benzoyl group.

Examples of alkoxycarbonyl group include methoxycarbonyl group, ethoxycarbonyl group, n-propoxycarbonyl group, isopropoxy carbonyl group, n-butoxycarbonyl group, isobutoxycarbonyl group, tert-butoxycarbonyl group, n-pentyloxycarbonyl group, isopentyloxy carbonyl group, hexyloxycarbonyl group, isohexyloxy carbonyl group, cyclohexyloxycarbonyl group, and benzyloxy carbonyl group. Preferred among them are methoxycarbonyl group and ethoxycarbonyl group.

Examples of acyloxy group include acetoxy group and benzoyloxy group.

Examples of α,β-unsaturated carboxylic acid used as raw materials for the reaction in the present invention include α,β-unsaturated carboxylic acid, α,β-unsaturated carboxylic acid ester, α,β-unsaturated amide, α,β-unsaturated aldehyde and α,β-unsaturated ketone. The method of the present invention is particularly effective when α,β-unsaturated carboxylic acid or α,β-unsaturated carboxylic acid ester is used as raw material.

The reason for this is not clear. It is assumed that in the α,β-unsaturated carboxylic acid and α,β-unsaturated carboxylic acid ester, reactivity in double bond is improved by activation of carbonyl group in reaction conditions according to the present invention.

The target β-mercaptocarboxylic acids can be obtained by selecting an α,β-unsaturated carboxylic acids corresponding to the target as reactive substrate.

Examples of α,β-unsaturated carboxylic acid include acrylic acid, methacrylic acid, crotonic acid, 2-methylcrotonic acid, 3-methylcrotonic acid, 2-pentenoic acid, fumaric acid, maleic acid, maleic anhydride, cinnamic acid, α-methylcinnamic acid, β-methylcinnamic acid, (2'-methyl)cinnamic acid, (3'-methyl)cinnamic acid, (4'-methyl)cinnamic acid, 2,3-dimethylcinnamic acid, 2,4-dimethylcinnamic acid, 3,4-dimethylcinnamic acid, (2'-hydroxy) cinnamic acid, (3'-hydroxy)cinnamic acid, (4'-hydroxy)cinnamic acid, (2',3'-dihydroxy)cinnamic acid, (2',4'-dihydroxy)cinnamic acid, (3',4'-dihydroxy)cinnamic acid, 4-methyl-2-pentenoic acid, 1-cyclohexene carboxylic acid, 1-cyclopentene carboxylic acid, 3-(2-furyl)acrylic acid, 2,5-dihydrothiophene-3-carboxylic acid, and 2-hexenoic acid.

Preferred among them are crotonic acid, acrylic acid, methacrylic acid, 2-pentenoic acid, fumaric acid, maleic acid, cinnamic acid and 4-methyl-2-pentenoic acid for industrial availability as raw materials.

Examples of α,β-unsaturated carboxylic acid ester include methyl acrylate, ethyl acrylate, propyl acrylate, isopropyl acrylate, butyl acrylate, methyl methacrylate, ethyl methacrylate, propyl methacrylate, isopropyl methacrylate, butyl methacrylate, methyl crotonate, ethyl crotonate, propyl crotonate, isopropyl crotonate, butyl crotonate, methyl 2-pentenoate, ethyl 2-pentenoate, propyl 2-pentenoate, isopropyl 2-pentenoate, butyl 2-pentenoate, dimethyl fumarate, diethyl fumarate, methyl fumarate, ethyl fumarate, dipropyl fumarate," isopropyl fumarate, butyl fumarate, dimethyl maleate, diethyl maleate, methyl maleate, ethyl maleate, dipropyl maleate, diisopropyl maleate, dibutyl maleate, methyl cinnamate, ethyl cinnamate, propyl cinnamate, isopropyl cinnamate, butyl cinnamate, methyl 4-methyl-2-pentenoate, ethyl 4-methyl-2-pentenoate, propyl 4-methyl-2-pentenoate, isopropyl 4-methyl-2-pentenoate, butyl 4-methyl-2-pentenoate and methyl 2,5-dihydrothiophene-3-carboxylate.

More preferred among them are methyl acrylate, methyl methacrylate, methyl crotonate, ethyl crotonate, methyl 2-pentenoate, ethyl 2-pentenoate, methyl 4-methyl-2-pentenoate, methyl cinnamate and ethyl cinnamate for industrial availability as raw materials.

Examples of α,β-unsaturated amide include acrylamide, N-methyl acrylamide, N-ethyl acrylamide, methacrylamide, N-methyl methacrylamide, N-ethyl methacrylamide, crotonic acid amide, N-methyl crotonic acid amide, N-ethyl crotonic acid amide, 3-methyl crotonic acid amide, maleic acid amide, cinnamic acid amide, N-methyl cinnamic acid amide, N-ethyl cinnamic acid amide, α-methyl cinnamic acid amide, N-methyl α-methyl cinnamic acid amide, and N-ethyl α-methyl cinnamic acid amide.

More preferred among them are acrylamide, methacrylamide, N-methyl methacrylamide, crotonic acid amide, 3-methyl crotonic acid amide, and cinnamic acid amide for industrial availability as raw materials.

Examples of α,β-unsaturated aldehyde include acrolein, crotonaldehyde, 3-methyl crotonaldehyde, 2-pentenaldehyde, fumaraldehyde, maleinaldehyde, cinnamaldehyde, α-methyl cinnamic aldehyde, (2'-methyl)cinnamic aldehyde, (3'-methyl)cinnamic aldehyde, (4'-methyl)cinnamic aldehyde, (2'-hydroxy)cinnamic aldehyde, (3'-hydroxy)cinnamic aldehyde, and (4'-hydroxy)cinnamic aldehyde.

More preferred among them are crotonaldehyde, 3-methyl crotonaldehyde, 2-pentenaldehyde, cinnamaldehyde for industrial availability as raw materials.

Examples of α,β-unsaturated ketone include methylvinyl ketone, ethylvinyl ketone, 3-pentene-2-one, 4-phenyl3-pentene-2-one, 3-hexene-2-one, 4-hexene-3-one, 1,3-diphenyl-2-propenone, 4-methyl-3-pentene-2-one, cyclopentene-2-one(=cyclopentenone), cyclohexene-2-one(=cyclohexenone), cycloheptene-2-one(=cycloheptenone), carvone, 2(5H)- furanone, 3-methyl-2(5H)- furanone, 4-methyl-2(5H)- furanone, 3,5-dimethyl-2(5H)-furanone, 5,6-dihydro-2H-pyran-2-one, α-methylene-γ-butyrolactone and 3-methyl-2-cyclohexene-1-one.

More preferred among them are methyl vinyl ketone, ethyl vinyl ketone, cyclopentenone, cyclohexenone and cycloheptenone for industrial availability as raw materials.

### [β-mercaptocarboxylic acids]

In β-mercaptocarboxylic acids obtained in the present invention, a hydrogen atom is bonded to the α-position of the α,β-unsaturated carboxylic acids and a mercapto group is bonded to the β-position. As described above, the target β-mercaptocarboxylic acids can be obtained by selecting α,β-unsaturated carboxylic acids corresponding to the target. A preferred example of β-mercaptocarboxylic acids is a compound which has α,β-unsaturated carboxylic acids described above as preferred with a mercapto group(-SH) bonded to the β-position.

### [solid acid catalyst]

The solid acid catalyst is an acid catalyst which is solid within a range of room temperature (20 to 30 °C) to the reaction temperature and is insoluble or hardly-soluble in the solvent used in the present invention.

Examples of solid acid catalyst include zeolite, acidic ion exchange resin, zirconium compound, double oxide supported silica gel(silica gel carrying nothing is excluded from catalysts usable in the present invention), hydrotalcite, attapulgite, kaolinite, alumina, hydroxyapatite, heteropoly acid supported catalyst, and other metal oxides. More preferred among them are zeolite, and acidic ion exchange resin for industrial availability. Specifically, those described in Japanese Patent Application Laid-Open No. 2007-100073, Japanese Patent Application Laid-Open No. 2007-99746, Japanese Patent Application Laid-Open No. 2003-305370, and Japanese Patent Application Laid-Open No. 2003-212803 can be used.

In the present invention, zeolite used as solid acid catalyst is a substance represented by general formula: M_{2/n}O·Al₂O₃·xSiO₂·yH₂O (M=Na, K, Ca, Ba, n is valence, x=2 to 10, y= 2 to 7) and has a three-dimensional network structure formed by (Al,Si)O₄ tetrahedrons form sharing vertexes with each other, in voids of which network structure alkali metal, alkali earth metal or water molecules are present.

Either natural zeolite or synthetic zeolite may be used in the present invention. Examples of natural zeolite include analcite, chabazite, erionite, natrolite, mordenite, clinoptilolite, heulandite, stilbite, laumontite and phelinite. Examples of synthetic zeolites include crystalline aluminosilicates such as A-type zeolite, X-type zeolite, Y-type zeolite, L-type zeolite and ZSM zeolite (product of Mobil Oil Incorporation), and metalloaluminosilicate or metallo-silicate containing different elements such as boron, iron, gallium, titanium, copper and silver.

Also, as zeolite, those which have been cation-exchanged with a cation species selected from alkali earth elements such as Mg,Ca and Sr, rare earth elements such as La and Ce, and VIII-Group elements such as Fe, Co, Ni, Ru, Pd and Pt, and those containing Zr, Hf, Cr, Mo, W or Th, can be used.

Particularly preferred zeolites are molecular sieves 3A(MS3A), molecular sieves 4A(MS4A), molecular sieves 5A(MS5A), molecular sieves 13X(13X), molecular sieves Y-54, molecular sieves LZ-15, and molecular sieves AZ-300.

A synthetic zeolite can be obtained by sufficiently mixing starting materials such as sodium silicate, sodium aluminate and silica gel, allowing crystals to deposit at 80 to 120 °C, washing with water to pH 9 to 12 and then conducting filtration.

Acidic ion exchange resin consists of an insoluble porous synthetic resin in which polymer matrix has a fine three-dimensional network structure having acidic ion exchange groups. Generally, it is called cation exchange resin. Examples of acidic ion exchange resin include those having a polymer matrix to which the sulfonate group or carboxylic acid group as ion exchange group is bonded, obtained by subjecting phenol and formaldehyde to condensation polymerization, or obtained by copolymerization of styrene or halogenated styrene and divinylbenzene.

Hydrotalcite is a multiple hydroxide represented by general formula M¹₈₋ₓM²ₓ(OH)₁₆CO₂·nH₂O. Compositions of the compound in which M¹ = Mg²⁺, Fe²⁺, Zn²⁺, Ca²⁺, Li²⁺, Ni²⁺, CO²⁺, or Cu²⁺ and M²=Al³⁺, Fe³⁺, or Mn³⁺ are known and examples thereof include those containing water molecules.

Examples of attapulgite include aqueous magnesium silicate having a fibrous structure produced by Floridin Co., a company in USA.

Kaolinite is an aqueous silicate salt mineral of aluminum, which is a clay mineral represented by formula: Al₂Si₂O₅(OH)₄.

Alumina is an oxide of aluminum. Examples thereof include a compound represented by formula: Al₂O₃.

Hydroxyapatite is a compound represented by formula: Ca₅(PO₄)₃(OH).

Examples of heteropoly acid supported catalyst include those obtained by allowing silica or the like to support a compound represented by formula [MX₁₂O_{40]}ⁿ⁻(X is a hetero element such as Si and P, M is a poly element such as Mo and W).

A double oxide is a high-order oxide compound consisting of two or more kinds of metal oxides, in which compound no presence of base ion as oxo-acid can be observed in its structure. Examples of double oxide include non-aqueous binary catalyst consisting of tungsten compound and zirconium compound.

Examples of other metal oxide include oxides of metals selected from a group consisting of Ge, Sn, Pb, B, Al, Ga, Mg, Zn, Cd, Cu, Fe, Mn, Ni, Cr, Mo, W, Ti, Zr, Hf, Y, La, Ce, Yb and Si.

### [hydrogen sulfides]

The "hydrogen sulfides" used in the present invention is hydrogen sulfide, sulfide salt or hydrosulfide salt. The hydrogen sulfide used here may be hydrogen sulfide gas derived from petroleum refinery, or synthetic hydrogen sulfide obtained by hydrogenating sulfur. The hydrogen sulfide may be introduced into a reactor in form of gas or in a state dissolved in reaction solvent used in the reaction. In a case where the hydrogen sulfide is supplied in form of gas, the hydrogen sulfide gas may be pressurized and supplied to the upper layer of the reaction solution or supplied through a gas disperser into the reaction solution. In a case where the hydrogen sulfide is dissolved in reaction solvent and supplied, the hydrogen sulfide gas is introduced into a mixer for dissolving the gas in the solvent.

Examples of metal for forming metal salts such as sulfide salt and hydrosulfide salt include alkali metals and alkali earth metals, specifically, lithium, sodium, potassium, magnesium and calcium. Examples of non-metals salts of sulfide salt and hydrosulfide salt include amine salt, alkyl amine salt and dialkyl amine salt, specifically, salts of ammonia, methylamine, ethylamine, dimethylamine, diethylamine, ethanol amine, N-methyl diethanol amine, diethanol amine, diisopropyl amine and diglycol amine.

Preferred among them are sodium salt, potassium salt, ammonium salt and amine salt having a 12 or less carbon atoms in consideration for industrial availability and costs.

Examples of sulfide salt include salts of lithium sulfide, sodium sulfide, potassium sulfide, magnesium sulfide, calcium sulfide, ammonium sulfide and methylamine sulfide. Examples of hydrosulfide salts include lithium hydrosulfide, sodium hydrosulfide, potassium hydrosulfide, ammonium hydrosulfide and methyl amine hydrosulfide.

Preferred among them are salts of sodium hydrosulfide, potassium hydrosulfide, ammonium hydrosulfide in consideration for industrial availability.

The preferred amount of hydrogen sulfides to be used is within a range of 0.7 to 7 equivalent amounts based on 1 mol of the α,β-unsaturated carboxylic acids, more preferably 1.0 to 5, most preferably 1.1 to 4.

If the amount of hydrogen sulfides is less than 0.7 equivalent amounts, a large amount of sulfide compounds is generated, which is not preferred. If the amount of hydrogen sulfides exceeds 7 equivalent amounts, the equipment for collecting unreacted hydrogen sulfides must be a large-scale one, which does not suit the practical process, although the yield of β-mercaptocarboxylic acids does not decrease.

In a case where the hydrogen sulfide gas is dissolved in solvent before reaction, it is preferred that the hydrogen sulfide gas be supplied and dissolved in to the reaction solution while the temperature of the reaction solution is kept at 10 °C or less.

Also, hydrogen sulfide gas generated by allowing a sulfide salt to react with a mineral acid such as hydrochloric acid and sulfuric acid may be used.

### [Reaction]

The reaction according to the present invention generates β-mercaptocarboxylic acids through reaction between α,β-unsaturated carboxylic acids and hydrogen sulfides. Reaction between crotonic acid and hydrogen sulfide can be illustrated as follows.

CH₃-CH=CH-COOH + H₂S →CH₃-CH(SH)-CH₂-COOH

The reaction is conducted in the presence of solvent compatible with water. There is no particular limitation on the amount of solid acid catalyst used in the reaction. The amount can be determined appropriately according to the scale of the reaction. A preferred range of the concentration of solid acid catalyst in the reaction solution is from 0.05 to 30 % by mass, more preferably 1 to 20 % by mass, most preferably, 3 to 10 % by mass. Here, the term "concentration in the reaction solution" means the mass percentage of the solid acid catalyst based on the total amount of α,β-unsaturated carboxylic acids, hydrogen sulfides, solvent and solid acid catalyst. In the present invention, the solid acid catalyst is used in a state dispersed in solvent or fixed to a fixed bed. The mass of the fixed bed is not included in the above total amount or in the mass of the catalyst.

If the amount of solid acid catalyst is less than 0.05 % by mass, the reaction proceeds too slowly, which is not practically suitable for industrial production. If the amount exceeds 30 % by mass, handleability becomes too low, which is inconvenient industrially.

There is no particular limitation on the method for allowing the solid acid catalyst to contact α,β-unsaturated carboxylic acids. For example, a batchwise manner in which the solid acid catalyst is added to a mixture solution containing α,β-unsaturated carboxylic acids in a reaction vessel and the mixture is stirred, or a continuous manner in which a mixture solution containing raw material α,β-unsaturated carboxylic acids is allowed to pass through a fixed bed reactor filled with the solid acid catalyst, may be employed.

In case of conducting the reaction in batchwise manner, the solid acid catalyst used there may be in form of powder, or in form of pellets or beads solidified with binder. In consideration for reactivity, it is more preferable to use powdery solid acid catalyst.

In case of conducting the reaction by using a fixed bed reactor in continuous manner, there is no limitation on the size and shape of the fixed bed reactor. A known reactor may be selected appropriately according to the scale of the reaction. In order to enhance production efficiency, two or more fixed bed reactors, connected in series or in parallel, may be used.

The fixed bed reactor is filled with a predetermined amount of solid acid catalyst and then placed for reaction. The fixed bed reactor filled with solid acid catalyst is heated to a predetermined temperature, and liquid or gas containing raw material α,β-unsaturated carboxylic acids and hydrogen sulfides are supplied through an inlet of the reactor at a predetermined flow rate. Inside the fixed bed reactor, the material is converted into β-mercaptocarboxylic acids and the reaction mixture containing the β-mercaptocarboxylic acids can be recovered through an outlet.

Liquid space velocity at which the liquid containing α,β-unsaturated carboxylic acids as raw material is supplied through an inlet of the reactor to flow in the reactor is 0.5 to 10 hours in terms of retention time in the reactor, preferably 1 to 8 hours.

### [solvent]

The solvent used in the reaction of the present invention is compatible with water. Also, the solvent is used as a mixture solvent with water. The solvent may be selected according to solubility, reactivity or the like of α,β-unsaturated carboxylic acids and hydrogen sulfides which serve as raw materials. There is no limitation on the type of the solvent used here.

Preferred examples of solvent used in the present invention include alcohols and polar aprotic solvents, both compatible with water.

Examples thereof include methanol, ethanol, isopropyl alcohol(IPA), propanol, acetone, dioxane, tetrahydrofuran(THF), N, N-dimethyl formamide(DMF), dimethyl sulfoxide(DMSO), N-methyl-2- pyrrolidone(NMP), 1-ethyl-2-pyrrolidone, 1-methyl-2-piperidone, 1-butyl-2-pyrrolidone,1-ethyl-2-piperidone,1,3-dimethylpiperidone, 1,3-dimethyl-3,4,5,6-tetrahydro-2(1H)-pyrimidinone, 1, 3-dimethyl-2-imidazolidinone(DMI), 1,3-diethyl-2-imidazolidinone, 2-pyrrolidone, γ-butyrolactone, γ-butyrolactam, formamide, N-methyl formamide, N-ethyl formamide, acetamide, N-methyl acetamide, N-ethyl acetamide, N,N-dimethyl acetamide, N,N-diethyl acetamide, N-methyl propane amide, N-ethyl propane amide, acetonitrile(ATN), ethyleneglycol, and diethyleneglycol dimethyl ether.

Moreover, a mixture solvent consisting of these solvents and water is particularly preferred for the purpose of enhancing the yield of β-mercaptocarboxylic acids. This is because α,β-unsaturated carboxylic acids serving as reactive substrate and sulfide salt and hydrosulfide salt which serve as sulfating agent are slightly soluble in organic solvent, which may cause decrease in the production yield. By adding an appropriate amount of water to the solvent, the solubility of these substances can be enhanced, which can accelerate the reaction.

Particularly preferred examples of alcohol include methanol, ethanol, and isopropyl alcohol(IPA).

Particularly preferred examples of polar aprotic solvent include tetrahydrofuran (THF), N,N-dimethyl formamide(DMF), 1,3-dimethyl-2-imidazolidinone(DMI) and N-methyl-2-pyrrolidone(NMP).

Also, it is more preferable that a polar aprotic solvent be used as solvent compatible with water. In case of using a mixture solvent containing water, it is preferable that the water content in the mixture solvent be in a range of 5 to 50 % by mass, more preferably 5 to 30 % by mass, most preferably 10 to 20 % by mass.

A preferred range of the amount of the solvent is from 200 to 3500 mass parts based on 100 mass parts of α,β-unsaturated carboxylic acids, more preferably 300 to 2000 mass parts, most preferably 400 to 1500 mass parts.

If the amount of the solvent is less than 200 mass parts, side reaction may readily proceed, which may cause a decrease in the production yield of β-mercapto carboxylic acids. If the amount of the solvent exceeds 3500 mass parts, side reaction can be suppressed and the yield of β-mercapto carboxylic acids is increased. In that case, however, the concentration of the reaction solution is diluted, which reduces productivity. Therefore, it is preferable that the amount of solvent be determined according to the balance between reaction yield and productivity.

### [reaction concentration]

A preferred concentration range of α,β-unsaturated carboxylic acids in the reaction solution is from 3 to 35 % by mass, more preferably 5 to 30 % by mass and most preferably 7 to 20 % by mass. If the reaction concentration is less then 3 % by mass, the reaction proceeds too slowly. If the concentration exceeds 35 % by mass, side reaction may cause a decrease in yield.

### [reaction temperature]

A preferred range of the reaction temperature is from 50 to 200 °C, more preferably 70 to 150 °C, most preferably 80 to 120 °C.

If the temperature is lower than 50 °C, the reaction proceeds too slowly. If the temperature exceeds 200 °C, side reaction may cause a decrease in the production yield. Since volatile gas is generated in the heating step, it is preferable that a closed-type reactor be used for the purpose of preventing organic solvent or hydrogen sulfide gas from going out of the reaction system.

### [reaction time]

The reaction time can be within a range of 0.1 to 12 hours. Generally, the reaction is completed in 2 to 8 hours. In a case where the reaction uses crotonic acids as raw material, generally, the conversion ratio of the raw material after about 4 hours becomes 95 % or more. Termination of the reaction may be determined by analyzing the conversion ratio of raw material and the concentration of β-mercapto carboxylic acids in the reaction solution.

### [reaction pressure]

A preferred range of the reaction pressure is from 0.1 to 3 MPa, more preferably 0.2 to 2 MPa, most preferably 0.4 to 1.5 MPa. If the pressure is less than 0.1 MPa, the reaction proceeds too slowly. If the pressure exceeds 3 MPa, management of reaction apparatuses becomes difficult, which is not preferred for safety.

### [purification]

Examples of isolating β-mercapto carboxylic acids from the reaction system after completion of the reaction include a method in which the whole reaction mixture is distilled after unnecessary solid catalyst is removed from the solvent through filtration, and a method where solid catalyst is filtered off, the remaining reaction mixture is separated into an organic phase and an an aqueous phase and then the organic phase is distilled. Either one of the methods may be employed in the present invention. In a case where two or more kinds of solvents having no compatibility with each other are used in the present invention, β-mercaptocarboxylic acids generated in the invention can be extracted by using a solvent having high affinity with β-mercaptocarboxylic acids and the thus extracted liquid is purified through distillation or crystallization, to thereby obtain β-mercaptocarboxylic acids as target compounds.

In a case where a single or two or more kinds of solvents capable of uniformly dissolving with each other are used, the above extraction method may be employed or the reaction mixture as produced may be purified through distillation.

In consideration for reducing the amount of waste solution generated from the reaction solution and shortening the industrial process, a purification method in which the reaction solution is directly distilled or recrystallized is preferred.

In case of purification through distillation, there is no particular limitation on distillation apparatuses used in the method. Any conventional distillation apparatus of batchwise manner, continuous-type or tower-type may be used. In case of mass distillation on an industrial scale, it is preferable to use a continuous type consisting of a heater, a distillation tower and a condenser.

Recrystallization method can be employed in a case where the target β-mercaptocarboxylic acid is a compound which is solid at room temperature. The method may be any of crystallization process by using a poor solvent in which β-mercaptocarboxylic acids show low solubility, neutralization crystallization by adding an acid or a base, and cooling crystallization by cooling the reaction solution.

By the above operations, β-mercaptocarboxylic acids can be produced with high production efficiency. The thus obtained β-mercapto carboxylic acids have high purity and are useful as an additive for polymer compound or as raw material for producing other reactive compounds.

### EXAMPLES

Hereinafter, the invention is explained in detail based on Examples, Comparative Examples and Reference Examples.

In the following Examples, numbers accompanied by "%" are based on mass unless otherwise specified. Further, the time point at which the temperature reaches a predetermined temperature is described as the reaction-starting time, and the pressure at that time point is described as the reaction-starting pressure. The reaction time means a time period measured from the time point when the temperature reached the predetermined temperature.

In the following Examples, each component was analyzed by high-performance liquid chromatography (hereinafter, simply referred to as "HPLC"). The analysis conditions are as follows.
Column: Shodex NN-814(length:20 cm, inner diameter: 0.5cm) manufactured by SHOWA DENKO K.K.,
Column temperature: 40° C,
Eluent: 0.1%H₃PO₄, 8mM-KH₂PO₄,
Flow rate: 1.5mL/ min,
Detection: RI,UV(detection wavelength210nm)

### Example 1:

To a 500ml-volume autoclave made of HASTELLOY C (registered trademark), N-methylpyrrolidone (NMP)(313g, manufactured by Junsei Chemical Co., Ltd.) was added, and crotonic acid (25.5g,0.3mol, manufactured by Tokyo Chemical Industry Co., Ltd) and molecular sieves4A(MS4A)(24g, powder, manufactured by Junsei Chemical Co., Ltd.) as solid acid catalyst were added thereto. Hydrogen sulfide(16.1 g,0.5 mol, 1.6 equivalent amounts based on crotonic acid, manufactured by Sumitomo Seika Chemicals Co., Ltd.) was allowed to be absorbed into the mixture while keeping the temperature in a range of 2 to 7 °C. Then the temperature was increased to 100 °C and kept at the temperature for 5 hours. The autoclave was cooled to 25 °C and the solution inside it was sampled. The sample was analyzed by HPLC and 3-mercaptobutanoic acid (25.6g, 0.2mol, yield: 72%) generated as a reaction product was confirmed. The conversion ratio of crotonic acid was 100 %. The reaction pressure was 0.6 MPa at the time when the reaction started and 0.4 MPa at the time when the reaction ended.

### Examples 2 to 11:

The reaction was conducted in the same manner as in Example 1 except that N-methyl-2-pyrrolidone(NMP), dimethylformamide(DMF), 1,3-dimethyl-2-imidazolidinone(DMI), tetrahydrofuran (THF), methanol, ethanol or isopropylalcohol(IPA) was used as solvent, and that the shape of molecular sieves 4A as solid acid catalyst and the amount of hydrogen sulfide were changed as shown in Table 1. The results are shown in Table 1.

**Table 1**

| Example No. | Zeolite type (Shape) | H₂Equivale nt amounts Based on crotonic acid | Solvent | Reaction pressure MPa (reaction start to end) | Conversion ratio of crotonic acid (%conv.) | 3-mercapto butanoic acid Yield (%) | 3- mercapto butanoic acid Selection ratio (%sel) |
|---|---|---|---|---|---|---|---|
| 1 | | 1.6 | NMP | 0.60→0.40 | 100 | 72 | 72 |
| 2 | | 3.9 | NMP | 0.78→0.74 | 100 | 82 | 82 |
| 3 | | 1.6 | DMF | 0.90→0.65 | 99 | 73 | 74 |
| 4 | MS4A | 3.2 | DMF | 1.58→1.18 | 100 | 85 | 85 |
| 5 | (powder) | 1.6 | DMI | 0.90→0.60 | 100 | 71 | 71 |
| 6 | | 2.5 | THF | 1.20→0.80 | 40 | 32 | 80 |
| 7 | | 1.0 | methanol | 0.92→0.75 | 63 | 40 | 63 |
| 8 | | 1.0 | ethanol | 0.91→0.73 | 70 | 48 | 69 |
| 9 | | 1.6 | IPA | 0.91→0.73 | 69 | 48 | 70 |
| 10 | MS4A (pellets) | 1.6 | NMP | 0.52→0.40 | 100 | 72 | 72 |
| 11 | MS4A (beads) | 1.6 | NMP | 0.55→0.43 | 100 | 72 | 72 |

### Example 12:

The reaction was conducted in the same manner as in Example 1 except that molecular sieves Y-54 (24 g, powder, manufactured by Union Showa K.K.) was used, that a mixture solvent of NMP (266g, manufactured by Junsei Chemical Co., Ltd) and distilled water (47g) was used as solvent, and that the reaction temperature was 115 °C. 3-mercaptobutanoic acid (27.0 g, 0.2 mol, yield: 76 %) generated as a reaction product was confirmed. The conversion ratio of crotonic acid was 100 %. The reaction pressure was 0.5 MPa at the time when the reaction started and 0.4 MPa at the time when the reaction ended.

### Example 13:

Except that molecular sieves LZ-15 (24 g, powder, manufactured by Union Showa K.K.) was used in place of molecular sieves Y-54, reaction was conducted in the same manner as in Example 7. Generation of 3-mercaptobutanoic acid (27.4 g, 0.2 mol, yield: 77%) was confirmed. The conversion ratio of crotonic acid was 100 %. The reaction pressure was 0.5 MPa when the reaction started and 0.4 MPa when the reaction ended.

### Example 14:

Except that molecular sieves AZ-300 (24 g, beads, manufactured by Union Showa K.K.) was used in place of molecular sieves Y-54, reaction was conducted in the same manner as in Example 7. Generation of 3-mercaptobutanoic acid (25.2 g, 0.2 mol, yield: 71%) was confirmed. The conversion ratio of crotonic acid was 98 %. The reaction pressure was 0.6 MPa when the reaction started and 0.5 MPa when the reaction ended.

### Example 15:

Except that molecular sieves 4A(MS4A)(24 g, powder, manufactured by Junsei Chemical Co., Ltd.) was used in place of molecular sieves Y-54, reaction was conducted in the same manner as in Example 7. Generation of 3-mercaptobutanoic acid (27.0 g, 0.2mol, yield: 76%) was confirmed. The conversion ratio of crotonic acid was 100 %. The reaction pressure was 0.5 MPa when the reaction started and 0.4 MPa when the reaction ended.

After completion of the reaction, molecular sieves 4A was subjected to filtration and the filtrate was distilled under reduced pressure, to thereby obtain the target 3-mercaptobutanoic acid (26.5 g, 0.22 mol, purity: 99 %, boiling point: 80 °C/1 Torr, recovery through distillation: 98 %, total yield: 74.5%).

### Examples 16-27:

Except that the types of α,β-unsaturated carboxylic acids were changed to the types as shown in Table 2, reaction was conducted in the same manner as in Example 1. The results are shown in Table 2.

**Table 2**

| Example No. | Zeolite type (Shape) | α,β-unsaturated carboxylic acid | Reaction pressure MPa (Reaction start to end) | Conversion ratio of α,β-unsaturated carboxylic acid (%conv.) | β-mercaptio carboxylic acids yield(%) | β-mercaptio carboxylic acids selection ratio(%sel) |
|---|---|---|---|---|---|---|
| 16 | | acrylic acid | 0.63→0.56 | 100 | 87 | 87 |
| 17 | | methyl acrylate | 0.54→0.47 | 100 | 87 | 87 |
| 18 | | 2-pentenoic acid | 0.45→0.42 | 96 | 67 | 70 |
| 19 20 | | methacrylic acid | 0.68→0.63 | 98 | 83 | 85 |
| | | fumaric acid | 0.50→0.50 | 95 | 72 | 76 |
| 21 | | maleic acid | 0.52→0.53 | 96 | 75 | 78 |
| 22 | MS4A(powder) | cinnamic acid | 0.48→0.45 | 99 | 82 | 82 |
| 23 | | 4-methyl | 0.56→043 | 96 | 71 | 74 |
| 24 | | cyclopentenone | 0.64→0.61 | 92 | 78 | 85 |
| 25 | | cyclohexenone | 0.61→0.57 | 96 | 77 | 80 |
| 26 | | crotonic acid amide | 0.65→0.62 | 71 | 63 | 88 |
| 27 | | Crotonaldehyde | 0.90→0.81 | 100 | 58 | 58 |

### Comparative Example 1:

To a 500ml-volume autoclave, methanol(313g, 9.8 mol, manufactured by Junsei Chemical Co., Ltd.) and methanesulfonic acid (10 g, 0.1 mol, manufactured by Tokyo Chemical Industry Co., Ltd.) were added, and methyl crotonate (28.9 g, 0.3 mol, manufactured by Tokyo Chemical Industry Co., Ltd) was added thereto. Hydrogen sulfide(18.1 g, 0.5 mol, manufactured by Sumitomo Seika Chemicals Co., Ltd.) was allowed to be absorbed into the mixture while keeping the temperature in a range of 2 to 7 °C. Then the temperature was increased to 130 °C and kept for 4.5 hours. The autoclave was cooled. The yield of methyl 3-mercaptobutanate was 0 %. The conversion ratio of methyl crotonate was 0 %. The reaction pressure was 0.6 MPa at the time when the reaction started and 0.6 MPa at the time when the reaction ended.

### Comparative Example 2:

To a 500ml-volume autoclave, NMP (313 g, 9.8 mol, manufactured by Junsei Chemical Co., Ltd.) was added and crotonic acid (25.5 g, 0.3 mol, manufactured by Tokyo Chemical Industry Co., Ltd) and silica gel (24 g, manufactured by Wako Pure Chemical Industries, Ltd.) as solid acid catalyst were added thereto. Hydrogen sulfide(18.1 g, 0.5 mol, 1.8 equivalent amounts based on crotonic acid, manufactured by Sumitomo Seika Chemicals Co., Ltd.) was allowed to be absorbed into the mixture while keeping the temperature in a range of 2 to 7 °C. Then the temperature was increased to 100 °C and kept for 5 hours. The autoclave was cooled to 25 °C and the solution inside it was sampled. The sample was analyzed by HPLC and 3-mercaptobutanoic acid (0.8 g, 0.01 mol, yield: 3 %) generated as a reaction product was confirmed. The conversion ratio of crotonic acid was 4 %. The reaction pressure was 0.6 MPa at the time when the reaction started and 0.6 MPa at the time when the reaction ended.

### Comparative Example 3:

To a 500ml-volume autoclave, toluene (313 g, 3.4 mol, manufactured by Junsei Chemical Co., Ltd.) was added, and crotonic acid (25.5 g, 0.3 mol, manufactured by Tokyo Chemical Industry Co., Ltd) and molecular sieves 4A (MS4A)(24 g, powder, manufactured by Junsei Chemical Co., Ltd.) as solid acid catalyst were added thereto. Hydrogen sulfide (18.1 g, 0.5 mol, 1.8 equivalent amounts based on crotonic acid, manufactured by Sumitomo Seika Chemicals Co., Ltd.) was allowed to be absorbed into the mixture while keeping the temperature in a range 2 to 7 °C. Then the temperature was increased to 100 °C and kept for 5 hours. The autoclave was cooled to 25 °C and the solution inside it was sampled. The sample was analyzed by HPLC and 3-mercaptobutanoic acid (0.4 g, 0.005 mol, yield: 2 %) generated as a reaction product was confirmed. The conversion ratio of crotonic acid was 3 %. The reaction pressure was 0.5 MPa at the time when the reaction started and 0.5 MPa at the time when the reaction ended.

### Comparative Example 4:

To a 500ml-volume autoclave, hexane (313 g, 3.6 mol, manufactured by Junsei Chemical Co., Ltd.) was added, and crotonic acid (25.5 g, 0.3 mol, manufactured by Tokyo Chemical Industry Co., Ltd) and molecular sieves 4A (24 g, powder, manufactured by Junsei Chemical Co., Ltd.) as solid acid catalyst were added thereto. Hydrogen sulfide (18.1 g, 0.5 mol, 1.8 equivalent amounts based on crotonic acid, manufactured by Sumitomo Seika Chemicals Co., Ltd.) was allowed to be absorbed into the mixture while keeping the temperature in a range 2 to 7 °C. Then the temperature was increased to 100 °C and kept for 5 hours. The autoclave was cooled to 25 °C. and the solution inside it was sampled. The sample was analyzed by HPLC and 3-mercaptobutanoic acid (0.8 g, 0.007 mol, yield: 2 %) generated as a reaction product was confirmed. The conversion ratio of crotonic acid was 3 %. The reaction pressure was 0.8 MPa at the time when the reaction started and 0.8 MPa at the time when the reaction ended.

### Reference Example:

According to description in Japanese Patent Application Laid-Open No. 2001-187778, a conventional method was tested.

An aqueous solution of 30 % by mass sodium hydrosulfide (187 g,1.0mol, prepared by dissolving the solid material manufactured by Junsei Chemical Co., Ltd. in distilled water) and sodium hydroxide (100 g, 2.50 mol, manufactured by Junsei Chemical Co., Ltd.) were mixed together to form a uniform solution. Then to the resultant aqueous solution, an aqueous solution of 19 % by mass sodium acrylate (247 g, 0.5mol, manufactured by Junsei Chemical Co., Ltd.) was added dropwise over 30 minutes while the solution was kept at a temperature range of 80 to 100 °C. Subsequently, the mixture was allowed to react at 80 to 100 °C for 6 hours. After the reaction, the reaction solution was added dropwise to 30% sulfuric acid (1400 g, prepared by dissolving 98% sulfuric acid manufactured by Junsei Chemical Co., Ltd in distilled water) in the nitrogen atmosphere over 1 hour while the temperature was kept at 5 °C. After the addition, 3 g of zinc powder was added thereto and the reaction mixture was treated at 40 °C. Sodium sulfide generated as byproduct was filtered off from the mixture and then extraction treatment with methyl-tert-butyl ether was conducted. The organic layer thus obtained was concentrated to obtain residue. By distilling the residue under reduced pressure, β-mercaptopropionic acid was obtained at total yield of 70 %.

This Reference Example had to include many steps after the reaction, such as neutralization using a large amount of sulfuric acid and treatment with zinc powder.

## Claims

1. A method for producing β-mercaptocarboxylic acids, comprising using as reaction solvent a solvent, which water can dissolve at an amount of 5% by mass or more at reaction temperature, in a reaction between α,β-unsaturated carboxylic acids selected from a group consisting of α,β-unsaturated carboxylic acid, α,β-unsaturated carboxylic acid ester, α,β-unsaturated amide, α,β-unsaturated aldehyde and α,β-unsaturated ketone and at least one of hydrogen sulfides selected from a group consisting of hydrogen sulfide, sulfide salt and hydrosulfide salt in the presence of a solid acid catalyst (excluding silica gel), wherein the β-mercaptocarboxylic acids to be produced corresponds to the starting materials.

2. The method for producing β-mercaptocarboxylic acids according to claim 1, wherein a mixed solvent of a solvent, which water can dissolve at an amount of 5% by mass or more at reaction temperature, and water is used as the reaction solvent.

3. The method for producing β-mercaptocarboxylic acids according to claim 2, wherein the water content of the reaction solvent is within a range of 5 to 50 % by mass.

4. The method for producing β-mercaptocarboxylic acids according to any one of claims 1 to 3, wherein the solvent, which water can dissolve at an amount of 5% by mass or more at reaction temperature, is a polar aprotic solvent or an alcohol.

5. The method for producing β-mercaptocarboxylic acids according to claim 4, wherein the polar aprotic solvent is one or more selected from a group consisting of tetrahydrofuran(THF), N,N-dimethyl formamide(DMF), 1,3-dimethyl-2-imidazolidinone(DMI) and N-methyl-2-pyrrolidone (NMP).

6. The method for producing β-mercaptocarboxylic acids according to claim 1, wherein the selected α,β-unsaturated carboxylic acid is α,β-unsaturated carboxylic acid or α,β-unsaturated carboxylic acid ester.

7. The method for producing β-mercaptocarboxylic acids according to claim 1 or 6, wherein the α,β-unsaturated carboxylic acid is acrylic acid, methacrylic acid, crotonic acid, 2-pentenoic acid, fumaric acid, maleic acid, maleic anhydride, cinnamic acid, α-methyl cinnamic acid, (2'-methyl)cinnamic acid, (3'-methyl)cinnamic acid, (4'-methyl)cinnamic acid, (2',3'-dimethyl)cinnamic acid, (2',4'-dimethyl)cinnamic acid, (3',4'-dimethyl)cinnamic acid, (2'-hydroxy)cinnamic acid, (3'-hydroxy)cinnamic acid, (4'-hydroxy)cinnamic acid, (2',3'-dihydroxy)cinnamic acid, (2',4'-dihydroxy)cinnamic acid, (3',4'-dihydroxy)cinnamic acid, 2-hexenoic acid or 4-methyl-2-pentenoic acid.

8. The method for producing β-mercaptocarboxylic acids according to claim 1 or 6, wherein the α,β-unsaturated carboxylic acid ester is methyl acrylate, ethyl acrylate, propyl acrylate, butyl acrylate, methyl methacrylate, ethyl methacrylate, propyl methacrylate, butyl methacrylate, methyl crotonate, ethyl crotonate, propyl crotonate, butyl crotonate, methyl 2-pentenate, ethyl 2-pentenate, propyl 2-pentenate, or butyl 2-pentenate.

9. The method for producing β-mercaptocarboxylic acids according to claim 1, wherein the α,β-unsaturated ketone is cyclopentenone, cyclohexenone, or cycloheptenone.

10. The method for producing β-mercaptocarboxylic acids according to claim 1, wherein the solid acid catalyst is at least one kind selected from a group consisting of acidic ion exchange resin, zirconium compound, zeolite, double oxide supported silica gel, hydrotalcite, attapulgite, kaolinite, alumina, hydroxyapatite, and heteropoly acid-supported catalyst.

11. The method for producing β-mercaptocarboxylic acids according to claim 1, wherein the solid acid catalyst is zeolite.

12. The method for producing β-mercaptocarboxylic acids according to claim 11, wherein the zeolite is molecular sieves 3A, molecular sieves Y-54, molecular sieves LZ-15, molecular sieves AZ-300, molecular sieves 4A, molecular sieves 5A, or molecular sieves 13X.

13. The method for producing β-mercaptocarboxylic acids according to claim 1, wherein the sulfide salt or hydrosulfide salt is alkali metal salt, alkali earth metal salt, ammonium salt, amine salt having 12 or less carbon atoms.

## Patentansprüche

1. Verfahren zum Herstellen von α,β-Mercaptocarbonsäuren, welches das Verwenden eines in Wasser in einer Menge von 5 Massen-% oder mehr bei Reaktionstemperatur lösbaren Reaktionslösungsmittels als Lösungsmittel in einer Reaktion zwischen α,β-ungesättigten Carbonsäuren, die aus der aus α,β-ungesättigter Carbonsäure, α,β-ungesättigtem Carbonsäureester, α,β-ungesättigtem Amid, α,β-ungesättigtem Aldehyd und α,β-ungesättigtem Keton bestehenden Gruppe ausgewählt sind, und mindestens einem Wasserstoffsulfid, der aus der aus Wasserstoffsulfid, Sulfidsalz und Hydrosulfidsalz bestehenden Gruppe ausgewählt ist, in Anwesenheit eines festen Säurekatalysators (ausschließlich Kieselgel) umfasst, wobei die herzustellenden β-Mercaptocarbonsäuren den Ausgangsmaterialien entsprechen.

2. Verfahren zum Herstellen von α,β-Mercaptocarbonsäuren nach Anspruch 1, wobei ein Mischlösungsmittel aus einem in Wasser in einer Menge von 5 Massen-% oder mehr bei Reaktionstemperatur lösbaren Lösungsmittel und Wasser als Reaktionslösungsmittel eingesetzt wird.

3. Verfahren zum Herstellen von α,β-Mercaptocarbonsäuren nach Anspruch 2, wobei der Wassergehalt des Reaktionslösungsmittels innerhalb eines Bereichs von 5 bis 50 Massen-% ist.

4. Verfahren zum Herstellen von α,β-Mercaptocarbonsäuren nach einem der Ansprüche 1 bis 3, wobei das in Wasser in einer Menge von 5 Massen-% oder mehr bei Reaktionstemperatur lösbare Lösungsmittel ein polares aprotisches Lösungsmittel oder ein Alkohol ist.

5. Verfahren zum Herstellen von α,β-Mercaptocarbonsäuren nach Anspruch 4, wobei das polare aprotische Lösungsmittel mindestens eines ist, das aus der Gruppe ausgewählt ist, die aus Tetrahydrofuran (THF), N,N-Dimethylformamid (DMF), 1,3-Dimethyl-2-imidazolidinon (DMI) und N-Methyl-2-pyrrolidone(NMP) besteht.

6. Verfahren zum Herstellen von α,β-Mercaptocarbonsäuren nach Anspruch 1, wobei die ausgewählte α,β-ungesättigte Carbonsäure eine α,β-ungesättigte Carbonsäure oder ein α,β-ungesättigter Carbonsäureester ist.

7. Verfahren zum Herstellen von α,β-Mercaptocarbonsäuren nach Anspruch 1 oder 6, wobei die α,β-ungesättigte Carbonsäure Acrylsäure, Methacrylsäure, Crotonsäure, 2-Pentensäure, Fumarsäure, Maleinsäure, Maleinsäureanhydrid, Zimtsäure, α-Methylzimtsäure, (2'-Methyl)zimtsäure, (3'-Methyl)zimtsäure, (4'-Methyl)zimtsäure, (2',3'-Dimethyl)zimtsäure, (2',4'-Dimethyl)-zimtsäure, (3',4'-Dimethyl)zimtsäure, (2'-Hydroxy)zimtsäure, (3'-Hydroxy)zimtsäure, (4'-Hydroxy)zimtsäure, (2',3'-Dihydroxy)zimtsäure, (2',4'-Dihydroxy)zimtsäure, (3',4'-Dihydroxy)zimtsäure, 2-Hexensäure oder 4-Methyl-2-pentensäure.

8. Verfahren zum Herstellen von α,β-Mercaptocarbonsäuren nach Anspruch 1 oder 6, wobei der α,β-ungesättigte Carbonsäureester Methylacrylat, Ethylacrylat, Propylacrylat, Butylacrylat, Methylmethacrylat, Ethylmethacrylat, Propylmethacrylat, Butylmethacrylat, Methylcrotonat, Ethylcrotonat, Propylcrotonat, Butylcrotonat, Methyl 2-pentenat, Ethyl-2-pentenat, Propyl-2-pentenat oder Butyl-2-pentenat ist.

9. Verfahren zum Herstellen von α,β-Mercaptocarbonsäuren nach Anspruch 1, wobei das α,β-ungesättigte Keton Cyclopentenon, Cyclohexenon oder Cycloheptenon ist.

10. Verfahren zum Herstellen von α,β-Mercaptocarbonsäuren nach Anspruch 1, wobei der feste Säurekatalysator mindestens einer ist, der aus der Gruppe ausgewählt ist, die aus saurem Ionenaustauscherharz, einer Zirconiumverbindung, Zeolith, Doppeloxid-geträgertem Kieselgel, Hydrotalcit, Attapulgit, Kaolinit, Aluminiumoxid, Hydroxyapatit und Heteropolysäure-geträgertem Katalysator besteht.

11. Verfahren zum Herstellen von α,β-Mercaptocarbonsäuren nach Anspruch 1, wobei der feste Säurekatalysator Zeolith ist.

12. Verfahren zum Herstellen von α,β-Mercaptocarbonsäuren nach Anspruch 11, wobei der Zeolith Molekularsieb 3A, Molekularsieb Y-54, Molekularsieb LZ-15, Molekularsieb AZ-300, Molekularsieb 4A, Molekularsieb 5A oder Molekularsieb 13X ist.

13. Verfahren zum Herstellen von α,β-Mercaptocarbonsäuren nach Anspruch 1, wobei das Sulfidsalz oder Hydrosulfidsalz ein Alkalimetallsalz, Erdalkalimetallsalz, Ammoniumsalz, Aminsalz mit 12 oder weniger Kohlenstoffatomen ist.

## Revendications

1. Procédé de production d'acides β-mercaptocarboxyliques, comprenant l'utilisation en tant que solvant de réaction d'un solvant, que l'eau peut dissoudre en une quantité de 5 % en masse ou plus à la température de la réaction, dans une réaction entre des acides carboxyliques α,β-insaturés choisis dans un groupe constitué d'un acide carboxylique α,β-insaturé, d'un ester d'acide carboxylique α,β-insaturé, d'un amide α,β-insaturé, d'un aldéhyde α,β-insaturé et d'une cétone α,β-insaturée et au moins l'un des sulfures d'hydrogène choisis dans un groupe constitué du sulfure d'hydrogène, d'un sel de sulfure et d'un sel d'hydrosulfure en présence d'un catalyseur acide solide (à l'exclusion d'un gel de silice), dans lequel les acides β-mercaptocarboxyliques devant être produits correspondent aux matériaux de départ.

2. Procédé de production d'acides β-mercaptocarboxytiques selon la revendication 1, dans lequel un solvant mixte d'un solvant, que l'eau peut dissoudre en une quantité de 5 % en masse ou plus à la température de la réaction, et d'eau est utilisé en tant que solvant de réaction.

3. Procédé de production d'acides β-mercaptocarboxyliques selon la revendication 2, dans lequel la teneur en eau du solvant de réaction est dans une plage de 5 à 50 % en masse.

4. Procédé de production d'acides β-mercaptocarboxyliques selon l'une quelconque des revendications 1 à 3, dans lequel le solvant, que l'eau peut dissoudre en une quantité de 5 % en masse ou plus à la température de la réaction, est un solvant aprotique polaire ou un alcool.

5. Procédé de production d'acides β-mercaptocarboxytiques selon la revendication 4, dans lequel le solvant aprotique polaire est un ou plusieurs composés choisis dans un groupe constitué du tétrahydrofuranne (THF), du N,N-diméthylformamide (DMF), de la 1,3-diméthyl-2-imidazolidinone (DMI) et de la N-méthyl-2-pyrrolidone (NMP).

6. Procédé de production d'acides β-mercaptocarboxyliques selon la revendication 1, dans lequel l'acide carboxylique α,β-insaturé choisi est un acide carboxylique α,β-insaturé ou un ester d'acide carboxylique α,β-insaturé.

7. Procédé de production d'acides β-mercaptocarboxyliques selon la revendication 1 ou 6, dans lequel l'acide carboxylique α,β-insaturé est de l'acide acrylique, de l'acide méthacrylique, de l'acide crotonique, de l'acide 2-penténoïque, de l'acide fumarique, de l'acide maléique, de l'anhydride maléique, de l'acide cinnamique, de l'acide α-méthylcinnamique, de l'acide (2'-méthyl)cinnamique, de l'acide (3'-méthyl)cinnamique, de l'acide (4'-méthyl)cinnamique, de l'acide (2',3'-diméthyl)cinnamique, de l'acide (2',4'-diméthyl)cinnamique, de l'acide (3',4'-diméthyl)cinnamique, de l'acide (2'-hydroxy)cinnamique, de l'acide (3'-hydroxy)cinnamique, de l'acide (4'-hydroxy)cinnamique, de l'acide (2',3'-dihydroxy)cinnamique, de l'acide (2',4'-dihydroxy)cinnamique, de l'acide (3',4'-dihydroxy)cinnamique, de l'acide 2-hexénoïque ou de l'acide 4-méthyl-2-penténoïque.

8. Procédé de production d'acides β-mercaptocarboxyliques selon la revendication 1 ou 6, dans lequel l'ester d'acide carboxylique α,β-insaturé est de l'acrylate de méthyle, de l'acrylate d'éthyle, de l'acrylate de propyle, de l'acrylate de butyle, du méthacrylate de méthyle, du méthacrylate d'éthyle, du méthacrylate de propyle, du méthacrylate de butyle, du crotonate de méthyle, du crotonate d'éthyle, du crotonate de propyle, du crotonate de butyle, du 2-penténate de méthyle, du 2-penténate d'éthyle, du 2-penténate de propyle ou du 2-penténate de butyle.

9. Procédé de production d'acides β-mercaptocarboxyliques selon la revendication 1, dans lequel la cétone α,β-insaturée est de la cyclopenténone, de la cyclohexénone ou de la cyclohepténone.

10. Procédé de production d'acides β-mercaptocarboxyliques selon la revendication 1, dans lequel le catalyseur acide solide est au moins un type choisi dans un groupe constitué d'une résine échangeuse d'ions acide, d'un composé à base de zirconium, d'une zéolite, d'un gel de silice supporté par un oxyde double, d'hydrotalcite, d'attapulgite, de kaolinite, d'alumine, d'hydroxyapatite et d'un catalyseur supporté par un hétéropolyacide.

11. Procédé de production d'acides β-mercaptocarboxyliques selon la revendication 1, dans lequel le catalyseur acide solide est une zéolite.

12. Procédé de production d'acides β-mercaptocarboxyliques selon la revendication 11, dans lequel la zéolite est constituée de tamis moléculaires 3A, de tamis moléculaires Y-54, de tamis moléculaires LZ-15, de tamis moléculaires AZ-300, de tamis moléculaires 4A, de tamis moléculaires 5A ou de tamis moléculaires 13X.

13. Procédé de production d'acides β-mercaptocarboxyliques selon la revendication 1, dans lequel le sel de sulfure ou le sel d'hydrosulfure est un sel de métal alcalin, un sel de métal alcalino-terreux, un sel d'ammonium, un sel d'amine comportant 12 atomes de carbone ou moins.
